# EUROPEAN PATENT APPLICATION

(11) **EP 3 680 815 A1**
(43) Date of publication of application: **15.07.2020**
(21) Application number: 19174346.7
(22) Date of filing: 14.05.2019
(51) Int. Cl.: G06K 9/00

(54) **TEMPERED GLASS PROTECTIVE FILM FOR ULTRASONIC FINGERPRINT RECOGNITION AND PREPARATION METHOD THEREOF**

(30) Priority: 08.01.2019 CN 201910016819
(71) Applicant: Shenzhen Taiji Opto-Elec Co., Ltd., Shenzhen, Guangdong (CN)
(72) Inventor: WEN, Zhiguang, Yichun City, Jiangxi Province (CN)
(74) Representative: Viering, Jentschura & Partner mbB Patent- und Rechtsanwälte

(57) **Abstract**

Disclosed are a tempered glass protective film for ultrasonic fingerprint recognition and a preparation method thereof. The tempered glass protective film comprises: a main film region (1) made of a tempered glass material, and a waveguide region (2) made of an ultrasonic high permeability material. The preparation method comprises the following steps: respectively preparing a tempered glass sheet and a waveguide sheet; opening a through hole on the tempered glass sheet; and fixing the waveguide sheet in the through hole on the tempered glass sheet to form a flat tempered glass protective film. Since the waveguide region is disposed, the ultrasonic wave is transmitted between the ultrasonic fingerprint sensor and the finger through the waveguide region, so that the echo energy received by the ultrasonic fingerprint sensor can be increased effectively, thereby enabling the ultrasonic fingerprint sensor to obtain a clear fingerprint image.

## Description

### BACKGROUND

### 1. Technical Field

The present invention relates to an electronic device protective film, in particular to a tempered glass protective film for ultrasonic fingerprint recognition and a preparation method thereof.

### 2. Description of Related Art

Electronic device protective films are widely used in smartphones, tablets, iPads, and so on to protect the screen of electronic devices. At present, the protective film for electronic devices is generally classified into a general protective film and a tempered glass protective film, both of which prevent the screen of the electronic device from being scratched. Compared with the ordinary protective film, the tempered glass protective film has the characteristics of high hardness and low toughness, so that it has better impact resistance, and can protect the screen when the electronic device falls to prevent screen damage.

As a biometric feature, fingerprints are unique and widely used to verify identity, and have been used in some electronic devices such as smartphones, tablets, and iPads to unlock these electronic devices. Fingerprint sensors are used to collect fingerprints, which can be divided into contact and non-contact types. The contact fingerprint sensor recognizes fingerprints through fingers pressing on the acquisition window. Non-contact fingerprint sensors currently have an ultrasonic fingerprint sensor that recognizes fingerprints by transmitting and detecting reflected ultrasonic waves. Since the ultrasonic wave has penetrability, it can penetrate the outer casing of the electronic device. Therefore, the ultrasonic fingerprint sensor can recognize the fingerprint even if it is disposed inside the outer casing of the electronic device, so that the fingerprint acquisition window can be arranged in the outer casing of the electronic device, and the appearance of the electronic device can be aesthetically improved.

It can be seen from the above analysis that compared with ordinary protective film and contact fingerprint sensor, the application of tempered glass protective film and ultrasonic fingerprint sensor in electronic devices such as smart phones, tablet computers, and iPads has significant advantages. However, tempered glass protective film and ultrasonic fingerprint sensor are difficult to be applied together on such electronic devices. The main reason is that the ability of ultrasonic waves to penetrate the tempered glass protective film is weak. Only a small part of the ultrasonic wave emitted by the ultrasonic fingerprint sensor can penetrate the tempered glass protective film to reach the finger. After being reflected by the finger, the ultrasonic waves that can reversely penetrate the tempered glass protective film back to the ultrasonic fingerprint sensor are less, which results in the inability to obtain a clear fingerprint image.

### SUMMARY

The object of the present invention is to provide a tempered glass protective film for ultrasonic fingerprint recognition, so as to solve the technical problem that the echo energy received by the ultrasonic fingerprint sensor is low when the tempered glass protective film is used in the electronic device, and the clear fingerprint image cannot be obtained.

In order to achieve the above object, the technical solution adopted by the present invention is as follows.

The present invention relates to a tempered glass protective film for ultrasonic fingerprint recognition, which comprises: a main film region (1) made of a tempered glass material; and a waveguide region (2) made of an ultrasonic high permeability material.

The waveguide region is correspondingly arranged according to different ultrasonic positions of an electronic device.

In one aspect, the main film region surrounds the periphery of the waveguide region.

In another aspect, the waveguide region is located at one edge of the tempered glass protective film.

Preferably, the waveguide region is rectangular, elliptical or circular, and the size of the waveguide region matches the size of the fingerprint region of finger.

Preferably, the ultrasonic high permeability material constituting the waveguide region is an organic glass, an epoxy resin or a transparent plastic.

Preferably, the ultrasonic high permeability material contains a photocurable component, and preferably, the photocurable component is a UV gel.

In some aspects, an adhesive layer is provided on the same side of the waveguide region and the main film region. Preferably, the glue of the adhesive layer contains AB glue.

In some aspects, the main film region (1) has: a first light-transmitting hole (3) corresponding to a camera, a second light-transmitting hole (5) corresponding to an optical sensor, and/or a sound-transmitting hole (4) corresponding to an earpiece.

The present invention relates to a preparation method of a tempered glass protective film for ultrasonic fingerprint recognition, wherein the tempered glass protective film comprises: a main film region (1) made of a tempered glass material, and a waveguide region (2) made of an ultrasonic high permeability material. The preparation method comprises the steps of respectively preparing a tempered glass sheet and a waveguide sheet; opening a through hole on the tempered glass sheet; and fixing the waveguide sheet in the through hole on the tempered glass sheet to form a flat tempered glass protective film.

The present invention relates to a preparation method of a tempered glass protective film for ultrasonic fingerprint recognition, wherein the tempered glass protective film comprises: a main film region (1) made of a tempered glass material, and a waveguide region (2) made of an ultrasonic high permeability material. The preparation method comprises the steps of: preparing a tempered glass sheet; opening a through hole on the tempered glass sheet; and injecting an ultrasonic high permeability material into the through hole, and forming the waveguide region through leveling, hot pressing, and natural drying.

Compared with the prior art, the present invention has at least the following beneficial effects.

Since the above-mentioned waveguide region is disposed, the ultrasonic wave is transmitted between the ultrasonic fingerprint sensor and the finger through the above-mentioned waveguide region, so that the echo energy received by the ultrasonic fingerprint sensor can be increased effectively, thereby enabling the ultrasonic fingerprint sensor to obtain a clear fingerprint image.

### BRIEF DESCRIPTION OF THE DRAWINGS

FIG. 1 is a schematic view illustrating the structure of a first embodiment of a tempered glass protective film.
FIG. 2 is a schematic view illustrating the structure of a second embodiment of a tempered glass protective film.
FIG. 3 is a schematic view illustrating the structure of a third embodiment of a tempered glass protective film.
FIG. 4 is a schematic view illustrating the structure of a fourth embodiment of a tempered glass protective film.

### DESCRIPTION OF THE EXEMPLARY EMBODIMENTS

The present invention will be further described below in conjunction with the drawings and embodiments.

The present invention provides a waveguide region in the tempered glass protective film. When used, the ultrasonic wave is transmitted between the ultrasonic fingerprint sensor and the finger through the above-mentioned waveguide region, so that the echo energy received by the ultrasonic fingerprint sensor can be increased effectively, thereby enabling the ultrasonic fingerprint sensor to obtain a clear fingerprint image.

The position of the waveguide region is determined according to the position of the ultrasonic fingerprint sensor in the corresponding electronic device. The size of the waveguide region should not be less than the size of the fingerprint region of finger, and it is preferable that the size of the waveguide region matches the size of the fingerprint region of finger. If the waveguide region is too large, the area of the main film region will become smaller, and the area of the screen where is shatterproof (the area corresponding to the main film region) is correspondingly reduced. If the waveguide region is too small, the ultrasonic wave reaching the edge of the fingerprint and the echo energy transmitting back to the sensor reflected by the fingerprint edge are small, and the edge of the fingerprint image is hence not clear enough, which may affect the fingerprint resolution. The shape of the waveguide region is preferably rectangular, elliptical or circular, with the elliptical shape being most preferable. The principle is that the ellipse is similar to the shape of the finger, and the utilization rate of the region is the highest, which can effectively reduce the proportion of the waveguide region.

### First embodiment

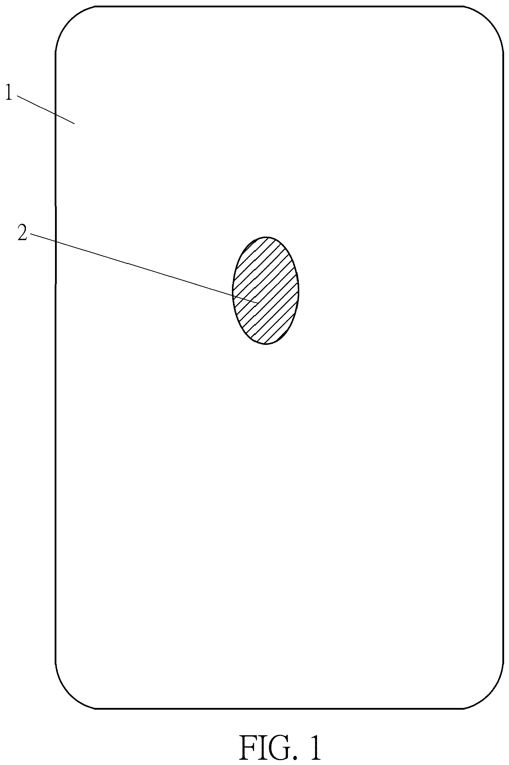

Referring to FIG. 1, the tempered glass protective film comprises: a main film region 1 made of a tempered glass material and a waveguide region 2 made of an ultrasonic high permeability material. The main film region 1 surrounds the periphery of the waveguide region 2, and more specifically, the waveguide region 2 is located in the middle of the tempered glass protective film. The shape of the waveguide region 2 is elliptical.

### Second embodiment

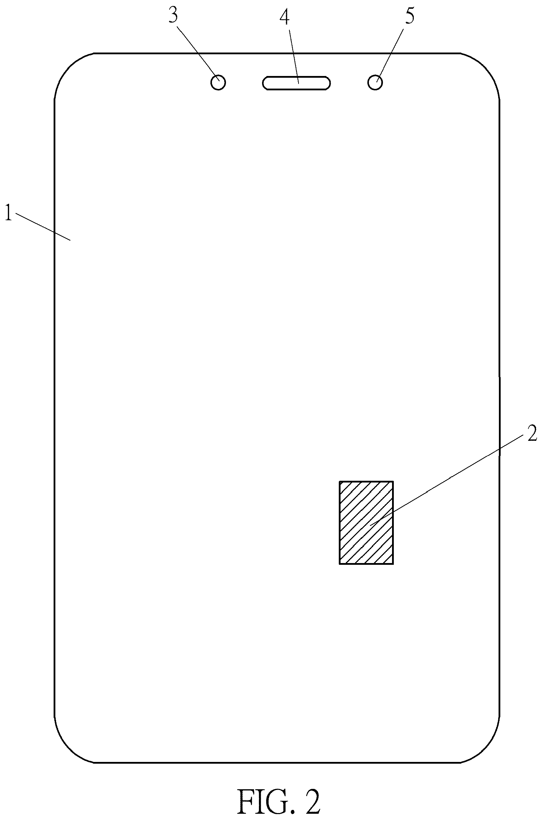

Referring to FIG. 2, the tempered glass protective film comprises: a main film region 1 made of a tempered glass material and a waveguide region 2 made of an ultrasonic high permeability material. The main film region 1 surrounds the periphery of the waveguide region 2, and more specifically, the waveguide region 2 is located at a position in the middle and at a lower right side of the tempered glass protective film, and has a rectangular shape. Further, the main film region 1 has a first light-transmitting hole 3 corresponding to a camera, a second light-transmitting hole 5 corresponding to an optical sensor, and a sound-transmitting hole 4 corresponding to an earpiece. The first light-transmitting hole 3 and the second light-transmitting hole 5 can improve the light transmittance, thereby improving the resolution of the image output by the camera and improving the sensitivity of the optical sensor. The sound-transmitting hole 4 enables the sound waves generated by the earpiece to be efficiently transmitted to the human ear.

### Third embodiment

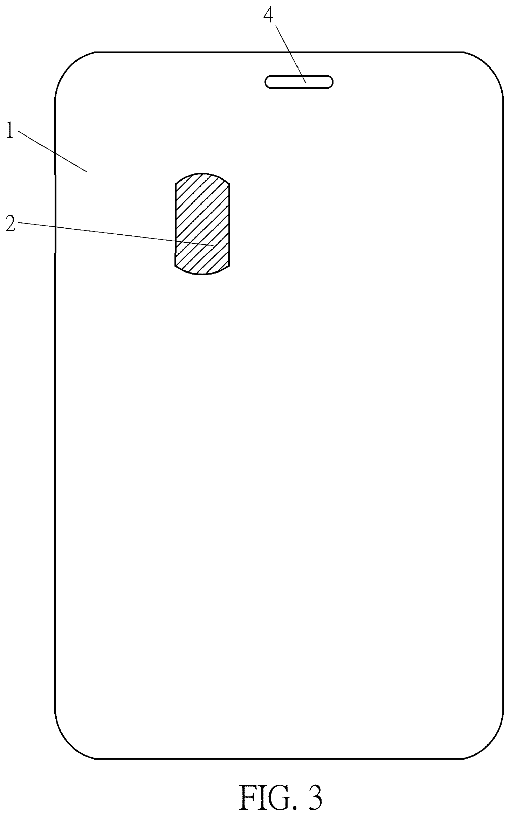

Referring to FIG. 3, the tempered glass protective film comprises: a main film region 1 made of a tempered glass material and a waveguide region 2 made of an ultrasonic high permeability material. The main film region 1 surrounds the periphery of the waveguide region 2, and more specifically, the waveguide region 2 is disposed at a position in the middle and the upper left side of the tempered glass protective film, and has a shape similar to a rectangle. Further, the main film region 1 has a sound-transmitting hole 4 corresponding to an earpiece. The sound-transmitting hole 4 enables the sound waves generated by the earpiece to be efficiently transmitted to the human ear.

### Fourth embodiment

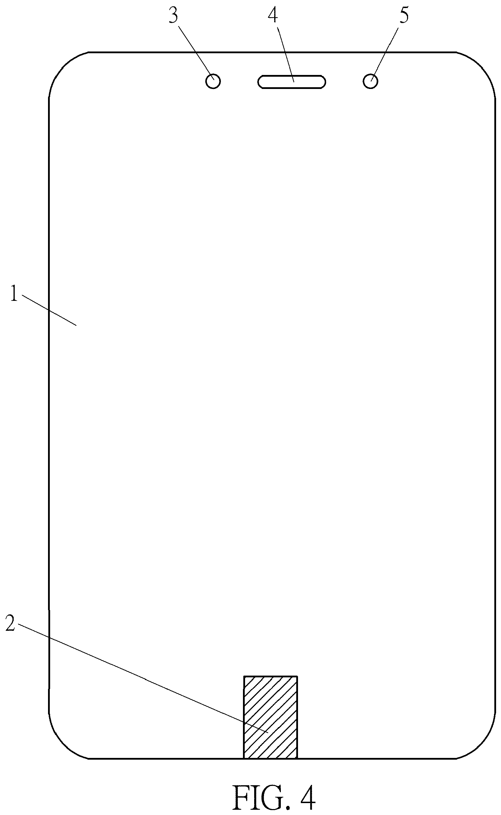

Referring to FIG. 4, the tempered glass protective film comprises: a main film region 1 made of a tempered glass material and a waveguide region 2 made of an ultrasonic high permeability material. The waveguide region 2 is located at a lower edge of the tempered glass protective film and has a rectangular shape. Further, the main film region 1 has a first light-transmitting hole 3 corresponding to a camera, a second light-transmitting hole 5 corresponding to an optical sensor, and a sound-transmitting hole 4 corresponding to an earpiece. The first light-transmitting hole 3 and the second light-transmitting hole 5 can improve the light transmittance, thereby improving the resolution of the image output by the camera and improving the sensitivity of the optical sensor. The sound-transmitting hole 4 enables the sound waves generated by the earpiece to be efficiently transmitted to the human ear.

As some embodiments, an adhesive layer is further provided on the same side of the waveguide region and the main film region, and the tempered glass protective film is bonded to the electronic device through the adhesive layer. The glue of the adhesive layer is preferably AB glue.

In the present invention, the material of the main film region 1 is a tempered glass. That is, it is the same as the existing tempered glass protective film. The material of the waveguide region 2 may be various ultrasonic high permeability materials such as an organic glass, an epoxy resin or a transparent plastic. As long as the ultrasonic permeability of the material is better than that of the tempered glass, it can be used as the material of the waveguide region 2. If the waveguide region 2 is disposed in the screen region of an electronic device, the material of the waveguide region 2 needs to meet the requirements of light transmission. That is, it needs to be a transparent material. If the waveguide region 2 is disposed in the non-screen region, the material of the waveguide region 2 may not necessarily be a transparent material. In some embodiments, the ultrasonic high permeability material contains a photocurable component, wherein the photocurable component is preferably UV glue (ultraviolet light curing adhesive), or a visible light curing material, and the ultrasonic high permeability material comprises a photocurable component. The ultrasonic high permeability material can be injected into the through hole in the tempered glass, and after the steps of leveling, hot pressing and natural drying, the waveguide region is formed, which is simpler to prepare.

The waveguide region 2 and the main film region 1 may be integrated into one by an adhesive process, or may be combined into one by a sintering process.

### Fifth embodiment

A preparation method of the above tempered glass protective film comprises the following steps.

Firstly, a tempered glass sheet and a waveguide sheet are separately prepared. In order to ensure the overall flatness of the tempered glass protective film, the thicknesses of the tempered glass sheet and the waveguide sheet should be equal.

Then, a through hole is opened on the tempered glass sheet, wherein the position of the through hole is determined according to the position of the ultrasonic fingerprint sensor in the corresponding electronic device, and should correspond to the ultrasonic receiving and transmitting end of the ultrasonic fingerprint sensor. The size of the through hole is equal to the size of the waveguide region, and the design principle thereof has been described in detail above, and will not be described herein.

Finally, the waveguide sheet is fixed in the through hole on the tempered glass sheet to form a flat tempered glass protective film. If the melting point of the waveguide sheet and the melting point of the tempered glass sheet are close to each other, the edge of the waveguide sheet and the edge of the through hole on the tempered glass can be fused together through the welding process, and the two can be combined into one. If the melting point of the waveguide sheet and the melting point of the tempered glass sheet are of a difference, and the edge of the waveguide sheet and the edge of the through hole on the tempered glass are bonded as one through glue process, wherein an adhesive is preferably, but not limited to, a two-component adhesive, and one component of the two-component adhesive is curing agent. The advantage of using a two-component adhesive can achieve rapidly hardening at room temperature, and the process is simpler and more efficient.

### Sixth embodiment

The other preparation method of the tempered glass protective film comprises the following steps.

Firstly, a tempered glass sheet is prepared.

A through hole is opened on the tempered d glass sheet;
After that, an ultrasonic high permeability material is injected into the through hole to form the waveguide region through the steps of leveling, hot pressing and natural drying. Preferably, the photocurable component, such as UV adhesive is included in the ultrasonic high permeability material.

The above detailed description of the present invention through specific embodiments is only limited to helping technical personnel in the field to understand the content of the present invention and cannot be understood as a limitation of the scope of protection of the present invention. All kinds of retouching and equivalent transformation of the above scheme by technicians in the field under the conception of the present invention shall be included in the protection scope of the present invention.

## Claims

1. A tempered glass protective film for ultrasonic fingerprint recognition, comprising:
a main film region (1) made of a tempered glass; and
a waveguide region (2) made of an ultrasonic high permeability material.

2. The tempered glass protective film for ultrasonic fingerprint recognition according to claim 1, wherein the waveguide region is correspondingly arranged according to different ultrasonic positions of an electronic device.

3. The tempered glass protective film for ultrasonic fingerprint recognition according to claim 1, wherein the main film region surrounds the waveguide region.

4. The tempered glass protective film for ultrasonic fingerprint recognition according to claim 1, wherein the waveguide region is located at one edge of the tempered glass protective film.

5. The tempered glass protective film for ultrasonic fingerprint recognition according to claim 1, wherein the waveguide region is rectangular, elliptical or circular, and the size of the waveguide region matches the size of the fingerprint region of finger.

6. The tempered glass protective film for ultrasonic fingerprint recognition according to claim 1, wherein the ultrasonic high permeability material constituting the waveguide region is an organic glass, an epoxy resin or a transparent plastic.

7. The tempered glass protective film for ultrasonic fingerprint recognition according to claim 6, wherein the ultrasonic high permeability material contains a photocurable component.

8. The tempered glass protective film for ultrasonic fingerprint recognition according to claim 7, wherein the photocurable component is UV glue.

9. The tempered glass protective film for ultrasonic fingerprint recognition according to claim 1, wherein an adhesive layer is provided on the same side of the waveguide region and the main film region.

10. The tempered glass protective film for ultrasonic fingerprint recognition according to claim 9, wherein the adhesive of the adhesive layer comprises AB glue.

11. The tempered glass protective film for ultrasonic fingerprint recognition according to claim 1, wherein the main film region (1) comprises:
a first light-transmitting hole (3) corresponding to a camera;
a second light-transmitting hole (5) corresponding to an optical sensor; and/or
a sound-transmitting hole (4) corresponding to an earpiece.

12. A preparation method of a tempered glass protective film for ultrasonic fingerprint recognition,
wherein the tempered glass protective film comprises a main film region (1) made of a tempered glass material and a waveguide region (2) made of an ultrasonic high permeability material;
wherein the preparation method comprises the following steps:
respectively preparing a tempered glass sheet and a waveguide sheet;
opening a through hole on the tempered glass sheet; and
fixing the waveguide sheet in the through hole on the tempered glass sheet to form a flat tempered glass protective film.

13. A preparation method of a tempered glass protective film for ultrasonic fingerprint recognition,
wherein the tempered glass protective film comprises a main film region (1) made of a tempered glass material and a waveguide region (2) made of an ultrasonic high permeability material;
wherein the preparation method comprises the following steps:
preparing a tempered glass sheet;
opening a through hole on the tempered glass sheet; and
injecting an ultrasonic high permeability material into the through hole, and forming the waveguide region through leveling, hot pressing, and natural drying.
